(19) Europäisches Patentamt
European Patent Office
Office européen des brevets

(11) **EP 4 670 711 A1**

(12) **EUROPEAN PATENT APPLICATION**
published in accordance with Art. 153(4) EPC

(43) Date of publication:
**31.12.2025 Bulletin 2026/01**

(21) Application number: **23923231.7**

(22) Date of filing: **24.02.2023**

(51) International Patent Classification (IPC):
**A61K 9/127** [(2025.01)]   **A61K 47/60** [(2017.01)]
**A61K 31/203** [(2006.01)]   **A61K 39/008** [(2006.01)]
**A61P 33/02** [(2006.01)]

(52) Cooperative Patent Classification (CPC):
**A61K 9/127; A61K 31/203; A61K 39/008;**
**A61K 47/60; A61P 33/02**

(86) International application number:
**PCT/BR2023/050062**

(87) International publication number:
**WO 2024/174011 (29.08.2024 Gazette 2024/35)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Rossi Bergmann, Bartira**
**22450-130 Rio de Janeiro (BR)**

(72) Inventors:
• **DE JESUS SOUSA BATISTA, Ariane**
**20735-080 Rio de Janeiro (BR)**

• **PEREIRA DA SILVA BEZERRA, Izabella**
**20261-065 Rio de Janeiro (BR)**
• **ALVES DE OLIVEIRA MELO JUNIOR, Otoni**
**21931-155 Rio de Janeiro (BR)**
• **LEONEL DE MATOS GUEDES, Herbert**
**20261-068 Rio de Janeiro (BR)**
• **ROSSI BERGMANN, Bartira**
**22450-130 Rio de Janeiro (BR)**

(74) Representative: **Hoyng Rokh Monegier B.V.**
**Rembrandt Tower, 30th Floor**
**Amstelplein 1**
**1096 HA Amsterdam (NL)**

(54) **LIPOSOME, METHOD FOR PREPARING A LIPOSOME, INTRANASAL COMPOSITION COMPRISING A LIPOSOME, METHOD FOR PREPARING AN INTRANASAL COMPOSITION, KIT AND USE OF THE COMPOSITION**

(57)     This invention relates to liposomes having mucopenetrating action and rates of incorporation of a retinoid, for example retinoic acid (RA), greater than those obtained in solid lipid nanoparticles (0.1%), thus increasing the uptake thereof via the nasal mucosa, and to the method for preparing a liposome. The present invention also relates to an intranasal composition, for example a vaccine, comprising said liposome, which is more effective than existing vaccines, the method for preparing same and the use thereof for preventing illnesses.

FIGURE 7

**Parasite Load**

**EP 4 670 711 A1**

**Description**

## FIELD OF THE INVENTION

This invention relates to liposomes with rates of retinoid incorporation, such as retinoic acid (RA) (also known as tretinoin, all-trans-retinoic acid, ATRA, or vitamin A acid) higher than previously obtained in solid lipid nanoparticles (0.1%), with mucopenetrating action in order to enhance uptake by the nasal mucosa, and its method of preparation.

The present invention is also related to an intranasal composition, such as a vaccine, comprising such liposome which has greater efficacy than currently existing vaccines, to its method of preparation and use for preventing diseases.

## BACKGROUND OF THE INVENTION

Cutaneous and visceral leishmaniasis are neglected diseases caused by Leishmania parasite transmitted to man by sandfly vectors. Cutaneous Leishmaniasis (CL) is the most common form and manifests itself by an ulcer at the site of sandfly bite which can spread throughout the body or cause permanent mutilations in the face). Despite being less frequent than CL, visceral leishmaniasis (VL) is the most severe form of disease. It mainly affects the liver, spleen and bone marrow and can be lethal if not treated properly.

In Brazil, the two forms of the disease occur mainly in humans and dogs, the latter being the main reservoirs and primary hosts of VL. Therefore, to contain increasing spread of the disease, dogs with positive serological diagnosis for leishmaniasis should not be treated with antileishmanial drugs used in humans and should be euthanized. There is still no approved vaccine for human use. In Brazil, there is currently only one licensed vaccine for veterinary use, Leish-Tec®. This can only be administered by injection (subcutaneously) to dogs from 4 months of age, enough time for puppies to acquire the disease and transmit it to other animals and humans through insect bites, since maternal antibodies have no protective function against leishmaniasis. Furthermore, its vaccine efficacy is only 71%. Therefore, even pre-vaccinated dogs can become infected. In this context, the search for an effective canine vaccine, with early protection against leishmaniasis, that is easy to administer to newborn dogs, so that it can be administered simultaneously with other vaccines in the canine vaccination schedule is highly desirable.

It has been demonstrated in different experimental models which, unlike classical subcutaneous vaccination, intranasal vaccination with *Leishmania amazonensis* antigens (LaAg) confers protective immunity against homologous and heterologous CL and VL infections in rodent models even in the absence of adjuvants (BEZERRA et al, 2018; DA SILVA-COUTO et al, 2015; LEAL et al, 2016; PINTO et al, 2003; 2004; PRATTI et al, 2016). Apparently, the vaccine protection mechanism is preferential induction of immune tolerance to deleterious Th2-type parasite antigens (BEZERRA et al, 2018). This is a novel strategy of vaccination against leishmaniasis, different from the classical injectable vaccines aiming to expand parasite-specific pro-inflammatory Th1 responses. For that, those vaccines are normally combined with strong adjuvants which have not proven sufficient to counteract the rapid Th2 response that tend to favor of the parasite not the host. Thus, the mucosal LaAg vaccine (intranasal, sublingual or oral) is more efficient than classical vaccines administered peripherally (topical, intradermal, subcutaneous or intramuscular) by **blocking** initial immune responses that favor penetration and establishment of leishmania in skin macrophages, thus enabling better performance of the innate and specific Th1 immune response in the rapid initial fight against infection, unlike LeishTec® whose immune response is directed at later stages, i.e. the intracellular amastigote form.

Dietary retinol (vitamin A) and its derivative, retinoic acid (RA), are crucial components in inducing oral tolerance to prevent our body's reaction to the high levels of vegetable and animal proteins present in food. RA leads to the expansion of suppressor FoxP3+ Treg cells in the mucosa, silencing both mucosal and peripheral immune responses (ERKELENS & MEBIUS, 2017), suggesting its great potential as an adjuvant for the intranasal LaAg vaccine. However, RA is known to have a strong irritant effect, which can cause irritation and discomfort during vaccine administration. Encapsulating RA in mucopenetrating nanosystems boosts mucosal permeation, enhances stability, enables slow release, and reduces mucosal irritation. Furthermore, it can also promote better uptake by lamina propria dendritic cells through their extensions, and consequent induction of FoxP3+ Treg cells. In addition to their application in leishmaniasis vaccines, these RA mucopenetrating nanosystems also have potential use in anti-allergy vaccines and in the treatment of inflammatory bowel diseases (e.g., food intolerance, celiac disease, Crohn's disease).

Previous studies have shown that the use of solid lipid nanoparticles (RA-NP) improves the efficacy of the intranasal LaAg vaccine compared to LaAg alone, demonstrated in different experimental models of leishmaniasis. For example, against CL by *L. amazonensis* in BALB/c mice (BEZERRA, 2012) and by *L. braziliensis* in hamsters (BEZERRA, 2016). And against VL, in mice infected with *L. infantum* compared with the commercial vaccine Leish-Tec®. LaAg+RA-NP was found to be more effective than Leish-Tec®, reducing the parasite loads in the spleen by 94% and in the liver by 91% compared to the non-immunized group, compared to 54 % and 82% reductions with Leish-Tec®, respectively.

Furthermore, the LaAg+RA-NP vaccine is also effective in newborn mouse against *L. infantum* infection, significantly reducing the parasite load in the spleen (75%) and liver (81%) compared to non-immunized animals. In this case, there was no comparison with Leish-Tec®, as the latter can only be administered to dogs over 4 months of age. It was also found that, like Leish-Tec®, LaAg+RA-NP does not induce anti-Leishmania antibodies that could interfere with the diagnosis of leishmaniasis (the control was carried out with unvaccinated infected animals) (BEZERRA et al., 2019). Another advantage of the liposomal system of the present invention compared to solid lipid nanoparticles (NP) is its greater RA encapsulation efficiency, which is much higher than that of NPs, whose efficiency is only 0.1% (CASTRO et al., 2009). Another advantage of the invention is that the nanoparticles are functionalized to amplify their mucopenetrating action (LAI; WANG; HANES, 2009), and consequently their adjuvanticity.

In this sense, the present invention proposes the development of a safe and innovative formulation consisting of mucopenetrating liposomes encapsulating RA to enhance its adjuvant effect in an intranasal canine vaccine against leishmaniasis, aiming for effective protection in both mature dogs and newborns, suitable for application within the canine vaccination schedule.

The study by Lima et al. (Alves; et al., 2007) reveals the encapsulation of isotretinoin, an isomer of RA (tretinoin), in liposomes. In that study, liposomes are prepared with phosphatidylcholine, cholesterol, and alpha-tocopherol. The present invention differs from that study by using RA, which is less toxic than isotretinoin (Diniz et al. 2002), and by presenting a different composition, requiring the presence of a PEGylated lipid, responsible for providing a liposome that, when used as an adjuvant in a vaccine, improves the efficacy of said vaccine; reduces the hydrophobicity of the particle surface; and by containing neither cholesterol nor alpha-tocopherol.

Furthermore, there is no suggestion that these liposomes disclosed in Lima et al. would be useful as vaccine adjuvants, including mucosal vaccines such as intranasal vaccines.

Document WO 2018/078053 describes lipid nanoparticles that can be used in vaccines. However, the combination of the teachings of WO 2018/078053 with Lima et al. will not arrive at the present invention, because the particles of WO 2018/078053 are not vaccine adjuvants and because, although they describe the use of cationic lipids, such lipids cannot be considered equivalent to the PEGylated lipid used in the present invention. Furthermore, WO 2018/078053 describes the use of encapsulated mRNA, while the present invention does not use mRNA, but rather antigens extracted from the parasite, and such antigens are not encapsulated.

Therefore, the liposomes of the present invention, as well as their preparation method, are novel and inventive compared to the prior art.

The paper by Bezerra (Bezerra et al., 2019) is the closest prior art to the present invention, as it deals with nanoencapsulated retinoic acid as an adjuvant for intranasal vaccines against leishmaniasis. The difference lies in the composition of the nanoparticle: while the article describes a nanoparticle comprising alpha-tocopherol, cholesterol, stearylamine, glycerol, EDTA, and polysorbate 80, the present invention contains only soy phosphatidylcholine, a PEGylated lipid, and retinoic acid. The results of the present invention are compared with the teachings of said paper, and the surprising technical effect of the present invention becomes evident.

Furthermore, there is no prior art teaching that indicates or suggests that a person skilled in the art should eliminate alpha-tocopherol, cholesterol, stearylamine, glycerol, EDTA, and polysorbate 80 from the nanoparticle and include soy phosphatidylcholine and a PEGylated lipid, and that doing so would have an improved effect.

Patent PI 0801417-5 describes a composition of solid lipid nanoparticles containing retinoids and lipophilic amines, intended for cosmetic application. The present invention differs from this patent at least for not requiring lipophilic amines and requires the inclusion of a PEGylated lipid. Furthermore, patent PI 081417-5 is completely silent regarding its use as an intranasal vaccine.

Application PI 0500726-7 describes vaccines administered mucosally where the adjuvant is the beta subunit of cholera toxin. The present invention differs from this application in that it does not use the beta subunit of cholera toxin and requires the inclusion of a PEGylated lipid.

Document WO 2006/110915 describes vaccine formulations against leishmania where the antigen is a protein from the K-39 group of *L. infantum.* This document also states that the vaccine can be formulated as a liposome, can be administered intranasally, and allows for the use of adjuvants. However, it does not mention that nanoencapsulated retinoids could be adjuvants, nor that the liposomes have a PEGylated lipid in their structure.

Document EP 2068918 describes vaccine compositions with a synthetic adjuvant, a glucopyranosyl lipid adjuvant. Although it mentions that the vaccine is effective against leishmaniasis and can be administered intranasally, this document does not mention that nanoencapsulated retinoids could be adjuvants, nor that the liposomes have a PEGylated lipid in their structure.

Therefore, it is clear that the present invention is novel and inventive compared to the prior art.

## SUMMARY OF THE INVENTION

It is an object of the present invention a liposome with high incorporation rate of a retinoid. Preferably, the liposome comprises:

a) at least one bilayer-forming lipid;
b) optionally at least one PEGylated lipid; and
c) at least one retinoid,

wherein the retinoid is incorporated in an amount of 0.1% to 20% w/w of the total liposome.

In a preferred embodiment, the lipid is phosphatidylcholine, the PEGylated lipid is DSPE-PEG, and the retinoid is all-trans retinoic acid.

An additional object of the present invention is a method for preparing a liposome with high retinoid incorporation. Preferably, the method comprises the steps of:

a) dissolving the bilayer-forming lipid, the optional PEGylated lipid, and the retinoid in chloroform;
b) forming a lipid film by eliminating the solvent;
c) hydrating the lipid film with a NaCl solution containing sucrose; and
d) extruding through polycarbonate membranes.

In a preferred embodiment, extrusion occurs under nitrogen pressure and the process is carried out protected from light. The process further comprises an optional step of lyophilizing the liposomes.

An additional object of the present invention is an intranasal composition comprising:

a) from $0.2\ \mu g/\mu L$ to $2.0\ \mu g/\mu L$ of a liposome comprising a retinoid, wherein the retinoid is incorporated in an amount of 0.1% to 20% w/w of the total liposome;
b) from $0.2\ \mu g/\mu L$ to $3.0\ \mu g/\mu L$ of at least one active ingredient; and
c) a carrier for intranasal administration.

In a preferred embodiment, the active ingredient is an antigen capable of providing immunity to an animal, such as a dog or a human. More preferably, the antigen is LaAg and the composition is a vaccine against leishmaniasis.

An additional object of the present invention is a method for preparing the intranasal composition comprising the liposome with high retinoid incorporation. Preferably, the method comprises the steps of:

a) obtaining at least one active ingredient; and
b) mixing the at least one active ingredient with the liposome in a carrier suitable for intranasal application.

In a preferred embodiment, the liposome and at least one active ingredient are in the form of a lyophilized powder to be reconstituted shortly before use.

Another object of the present invention is a kit comprising:

a) a lyophilized powder for reconstitution; and
b) a solvent;

wherein the lyophilized powder comprises:

1) a liposome-incorporated retinoid, wherein the retinoid is incorporated in an amount of 0.1% to 20% w/w of the total liposome; and
2) at least one active ingredient.

Another object of the present invention is the use of the intranasal composition for the prevention of leishmaniasis.

## BRIEF DESCRIPTION OF THE FIGURES

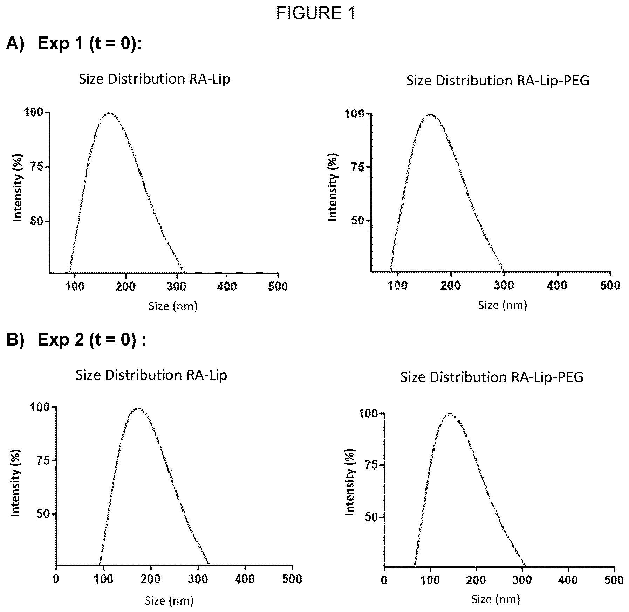

**Figure 1: Size polydispersity immediately after reconstitution in PBS.** Lyophilized RA-Lip and RA-LipPEG liposomes were resuspended in PBS and their size was then measured by DLS (t = 0) in two independent experiments (A and B).

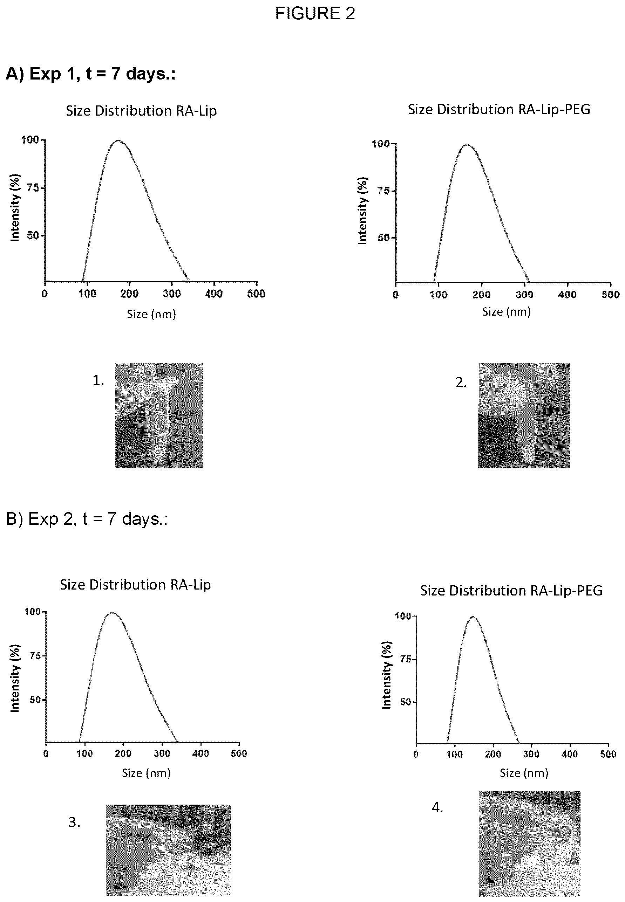

**Figure 2: Size polydispersity seven days after reconstitution in PBS.** Lyophilized RA-Lip and RA-LipPEG liposomes were resuspended in PBS, allowed to stand for 7 days at 4°C, and their size was measured by DLS (t = 7) in two independent experiments (A and B); RA-Lip suspension (1) and RA-Lip-PEG suspension (2) 7 days after reconstitution and RA-Lip suspension (3) and RA-Lip-PEG suspension (4) 7 days after reconstitution.

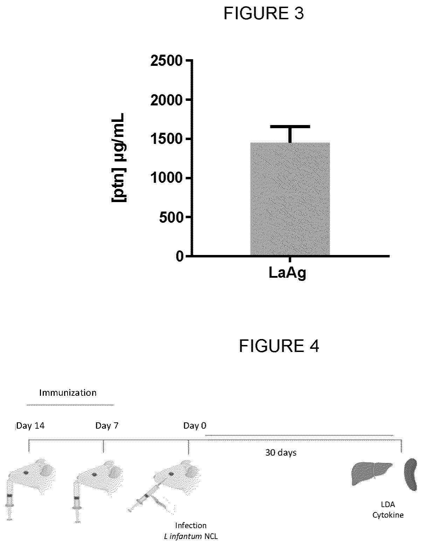

**Figure 3: Protein assay in the LaAg vaccine.** Lyophilized LaAg were resuspended in PBS, and protein quantification was performed using a commercial BCA kit (Sigma-Aldrich).

**Figure 4. Experimental protocol for vaccination and challenge with VL.** BALB/c mice were vaccinated with two intranasal doses seven days apart. Seven days after the second dose, they were infected with $10^7$ *L. infantum* promastigotes via i.v. (intraorbital) route. On day 30 of infection, the animals were euthanized, and their spleens and livers were removed for parasite load analysis by LDA.

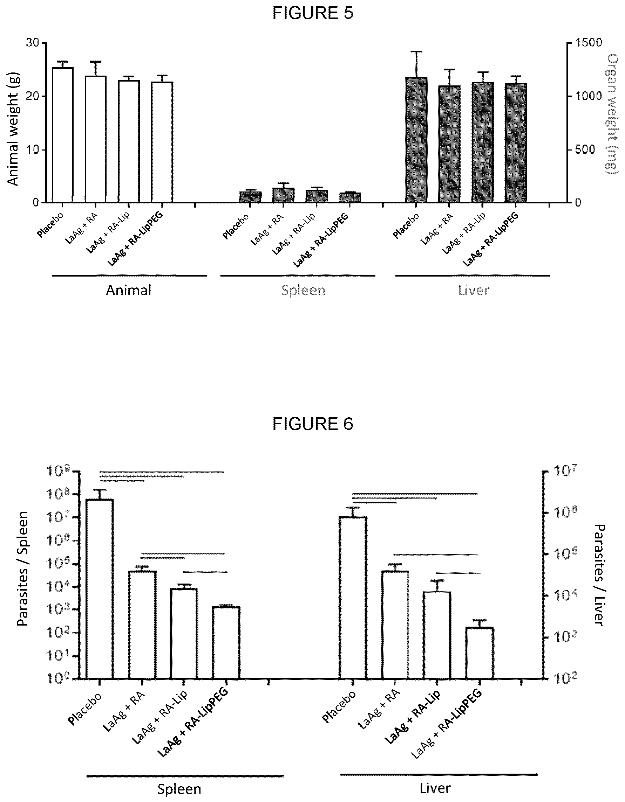

**Figure 5: Vaccination does not affect body and organ weight gain after infection in mice.** BALB/c mice were vaccinated with two intranasal doses of 20 µL of PBS (Placebo); LaAg; LaAg +RA-Lip; LaAg + RA-LipPEG containing 10 µg LaAg and 10 µg RA in the adjuvants, 7 days apart. Seven days after the last dose, the animals were infected with $10^7$ *L. infantum* promastigotes via i.v. (intraorbital) route. On day 30 of infection, the animals, their spleens, and livers were weighed. Means $\pm$ SD (n=5).

**Figure 6: Efficacy of the LaAg vaccine and its RA adjuvants (free RA, RA-Lip, and RA-LipPEG) in mice challenged with *L. infantum*.** BALB/c mice were vaccinated with two intranasal doses of 20 µL of PBS (Placebo); LaAg; LaAg + RA-Lip; LaAg + RA-LipPEG containing 10 µg LaAg and 10 µg RA in the adjuvants, 7 days apart. Seven days after the last dose, the animals were infected with $10^7$ *L. infantum* promastigotes via i.v. (intraorbital) route. The spleen and liver were weighed, macerated in their entirety, and the parasite load was assessed by LDA. Means $\pm$ SD (n=5). The connecting bars represent statistically significant differences between groups (p<0.05).

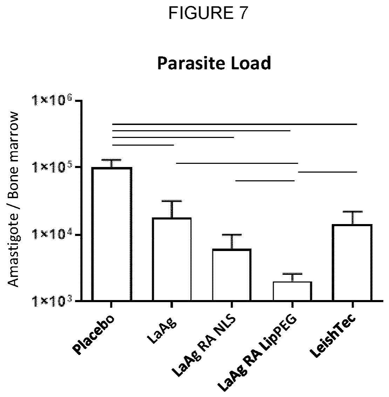

**Figure 7: Efficacy of the LaAg vaccine and its adjuvants compared to the commercial LeishTec® vaccine in mice challenged with *L. infantum*.** BALB/c mice were vaccinated with two intranasal doses of 20 µL of PBS (Placebo), LaAg; LaAg + NLS; LaAg + RA-LipPEG containing 10 µg LaAg and 10 µg RA in the adjuvants. Positive controls received 3 doses of the Leish-Tec vaccine subcutaneously (10 µg A2 + 50 µg saponin/dose = 100 uL each). Seven days after the last dose, the animals were infected on the same day with $10^7$ *L. infantum* promastigotes via i.v. (intraorbital) route. On day 30 after infection (PID=30), bone marrow was removed from the femurs, and the parasite load was assessed by LDA. Means $\pm$ SD (n=5). The connecting bars represent statistically significant differences between groups p<0.05.


## DETAILED DESCRIPTION OF THE INVENTION

The following description is intended solely to illustrate some of the numerous embodiments of the invention and should not be construed as limiting the scope of the present invention.

The liposome according to the present invention has a high incorporation of a retinoid when compared to nanoparticles for intranasal administration described in the prior art, such as those described by Bezerra (Bezerra et al., 2019). Preferably, the liposome comprises:

a) at least one bilayer-forming lipid;
b) optionally at least one PEGylated lipid; and
c) at least one retinoid, wherein the retinoid is incorporated in an amount of 0.1% to 20% w/w of the total liposome.

The at least one bilayer-forming lipid can be chosen from among the lipids used to produce unilamellar liposomes. Non-limiting examples of these lipids or phospholipids are selected from the group of sterols, sphingolipids and/or glycerolipids, in particular selected from the group comprising cholesterol, sphingomyelins, ceramides, phosphatidylcholines, phosphatidylethanolamines, phosphatidylserines, diacylglycerols, and phosphatidic acids containing one (lyso-) or two (diacyl-) saturated or unsaturated fatty acids with more than 4 carbon atoms and up to 28 carbon atoms.

Fatty acids comprising between 4 and 28 carbon atoms are, for example, saturated linear alkanecarboxylic acids, preferably with an even number of carbon atoms, such as between 12 and 26 carbon atoms, for example lauric, myristic, palmitic, stearic, arachidic or behenic acid, or unsaturated linear alkenecarboxylic acids, preferably with an even number of carbon atoms, such as between 12 and 26 carbon atoms, and one, two or more, preferably up to six double bonds in trans or preferably cis configuration, for example oleic acid, linoleic acid, alpha-linoleic acid, arachidonic acid or erucic acid.

Specifically, the lipid is selected from the group comprising phosphatidic acid (PA), phosphatidylglycerol (PG), phosphatidylethanolamine (PE) and phosphatidylserine (PS), phosphatidylcholine (PC), dipalmitoylphosphatidylcholine (DPPC), distearoylphosphatidylcholine (DSPC), dimyristoylphosphatidylcholine (DMPC), dipalmitoylphosphatidylglycerol (DPPG), distearoylphosphatidylglycerol (DSPG), dimyristoylphosphatidylglycerol (DMPG), dipalmitoylphosphatidic acid (DPPA); dimyristoylphosphatidic acid (DMPA), distearoylphosphatidic acid (DSP A), dipalmitoylphosphatidylserine (DPPS), dimyristoylphosphatidylserine (DMPS), distearoylphosphatidylserine (DSPS), dipalmitoylphosphatidylethanolamine (DPPE), dimyristoylphosphatidylethanolamine (DMPE), distearoylphosphatidylethanolamine (DSPE), dioleylphosphatidylethanolamine (DOPE), and combinations thereof.

The at least one optional PEGylated lipid is typically prepared from the derivatization of the polar head of a diacylglycerolphospholipid with PEG. In particular, any lipid mentioned above that can be derivatized with PEG is useful in the present invention. Thus, non-limiting examples of PEGylated lipids include PEG-distearoylphosphatidylethanolamine (DSPE-PEG), PEG-dioleylphosphatidylethanolamine (DOPE-PEG), PEG-ceramides (CER-PEG), and combinations thereof. The most preferred compound of the present invention is DSPE-PEG, especially DSPE-PEG$_{2000}$.

The at least one retinoid useful in the present invention is preferably chosen from first, second, or third generation retinoids. In particular, the retinoids according to the present invention are chosen from the group comprising retinoic acid (especially all-trans retinoic acid - tretinoin), retinyl palmitate, retinyl ester, retinol, retinal, isotretinoin, alitretinoin, etretinate, acitretin, tazarotene, bexarotene, adapalene, and combinations thereof.

The liposomes according to the present invention are prepared by the lipid film hydration technique. This technique occurs without the presence of light and comprises the steps of:

> a) dissolving the at least one bilayer-forming lipid, the at least one optional PEGylated lipid, and the at least one retinoid in chloroform;
> b) forming a lipid film by eliminating the solvent;
> c) hydrating the lipid film with a NaCl solution containing sucrose; and
> d) extruding through polycarbonate membranes;

The dissolution step comprises from 85% w/w to 99.5% w/w of the at least one bilayer-forming lipid, from 7% w/w to 12% w/w of the at least one PEGylated lipid, and from 0.5% w/w to 6% w/w of the at least one retinoid.

Once dissolved, the solution is transferred to a round-bottom flask and placed in a vacuum evaporator to remove the solvent and form the lipid film.

The film is then hydrated with a 0.9% NaCl solution containing sucrose as a cryoprotectant until it completely detaches from the walls of the flask. The ratio of moles of sucrose to moles of lipid varies from 1:0.5 to 1:20, preferably from 1:1 to 1:10.

Finally, the samples are extruded through polycarbonate membranes under nitrogen pressure to form uniform, unilamellar liposomes.

The resulting liposomes can be lyophilized for later use.

The present invention further describes an intranasal composition comprising the liposomes prepared above. Preferably, the intranasal composition comprises:

> a) from 0.2 $\mu$g/$\mu$L to 2.0 $\mu$g/$\mu$L of a liposome comprising a retinoid, wherein the retinoid is incorporated in an amount of 0.1% to 20% w/w of the total liposome;
> b) from 0.2 $\mu$g/$\mu$L to 3.0 $\mu$g/$\mu$L of at least one active ingredient; and
> c) a carrier for intranasal administration.

Since the liposome of the present invention is a mucopenetrating nanosystem that enhances the permeation of an active ingredient into the mucosa while not causing mucosal irritation, any active ingredient that can be delivered intranasally is within the scope of the present invention.

Any antigen or combination of antigens can be used in the present invention as an active ingredient. An antigen is understood to be a substance capable of eliciting a specific immune response in an individual. The antigen may have a protein characteristic and may be derived from the virus, bacteria, parasite, or animal cell, including a human cell, that one wishes to stimulate and/or prevent immunity. The present invention primarily aims at preventing the immune response, as in the case of autoimmune diseases and allergies, or any immunopathology, including leishmaniasis. In addition to the above, examples of suitable antigens within the scope of the present invention include recombinant proteins, DNA, and RNA.

In a preferred embodiment, the active ingredient is an antigen capable of stimulating and/or preventing immunity in an animal, such as a dog or a human. More preferably, the antigen is derived from an organism selected from the group comprising *Leishmania donovani, L. pifanoi, Leishmania amazonensis, Leishmania mexicana, Leishmania tropica, Leishmania braziliensis, Leishmania guyanensis,* and *Leishmania infantum.* Most preferably, the antigen is LaAg, resulting from the lysis of *L. amazonensis* promastigotes.

If the active ingredient is LaAg or another antigen derived from Leishmania, the present invention further describes the use of this composition (vaccine) for the prevention of leishmaniasis.

Active ingredients capable of preventing allergies, autoimmune diseases, inflammatory diseases, or transplant rejection are also considered within the scope of the present invention.

The carrier for intranasal administration is any carrier known in the art and used by a person skilled in the art.

The method for preparing the intranasal composition comprises the steps of:

a) obtaining at least one active ingredient; and

b) mixing the at least one active ingredient with the liposome in a carrier suitable for intranasal application.

Obtaining the active ingredient can be as simple as purchasing a product or more complex, involving cell culture, lysis, and separation.

The present invention also describes a kit comprising the composition in the form of a lyophilized powder and a solvent for reconstitution. Specifically, the kit comprises:

a) a lyophilized powder for reconstitution; and

b) a solvent;

wherein the lyophilized powder comprises:

1) a liposome-incorporated retinoid, wherein the retinoid is incorporated in an amount of 0.1% to 20% w/w of the total liposome; and

2) at least one active ingredient.

The solvent for reconstitution can be any solvent commonly used in the art, preferably an aqueous solvent. The kit also includes instructions for preparing and administering the composition.

**Examples**

Example 1 - Liposome Preparation

Conventional or pegylated liposomes containing retinoic acid were obtained through the lipid film hydration technique. To prepare the liposomes, soy phosphatidylcholine (PC), 2-distearol-sn-glycero-3-phosphoethanolamine-N-[amino(polyethylene glycol)-2000] (DSPE-PEG$_{2000}$), and retinoic acid (RA - All-trans-Retinoic acid 97% - Acrós Organics) were weighed as described in Table 1, dissolved in 1.5 mL of chloroform, and transferred to a round-bottom flask.

The "blank" liposomes were prepared in the same manner without the addition of the drug.

Table 1: Formulation Composition.

|  | PC | DSPE-PEG$_{2000}$ | RA |
| --- | --- | --- | --- |
| Blank Liposome (Lip) | 240 mg | -- | -- |
| Blank Liposome PEG (LipPEG) | 216 mg | 24 mg | -- |
| Liposome with RA (RA-Lip) | 240 mg | -- | 6 mg |
| Liposome PEG with RA (RA-LipPEG) | 216 mg | 24 mg | 6 mg |

The solution was placed in a rotary evaporator under vacuum for 2 h to eliminate residual solvent and form a lipid film. The lipid film was then hydrated with 2 mL of a 0.9% NaCl solution containing sucrose as a cryoprotectant for 30 min, until the film completely detached from the flask wall. Finally, the samples were extruded through 200 nm polycarbonate membranes (Whatman) under nitrogen pressure to form uniform, unilamellar liposomes. Throughout the procedure, the samples were protected from light.

Immediately after preparation, a 50 μL sample was diluted in 950 μL of ultrapure water to determine the mean diameter (MD), polydispersity index (PdI), and zeta potential (PZ) of the liposomes using the dynamic light scattering technique (DLS, Malvern Instruments). The samples were then frozen at -20°C and lyophilized for 20 hours. Liposome size was also assessed using the same technique after reconstitution of the liposomes in PBS to assess their stability. The RA concentration in the liposomes was analyzed by a spectrophotometer at 350 nm. The retinoic acid standard and the liposome were dissolved in ethanol p.a. for analysis, and the standard curve was prepared with a range of 0.5 to 8 μg/mL.

Example 2 - Determination of the Average Size and Zeta Potential of Liposomes

Immediately after preparation, the formulations were analyzed for average size and zeta potential, fundamental physicochemical parameters for quality control of the system. Table 2 shows the values for particle diameter, homogeneity, and zeta potential before and after lyophilization.

Table 2 - Determination of Liposome Diameter and Zeta Potential.

| | Pre-lyophilization | | | Post-lyophilization | | |
|---|---|---|---|---|---|---|
| | DM (nm) | Pdl | PZ (mV) | DM (nm) | Pdl | PZ (mV) |
| Lip | 152.1 | 0.075 | -1.26 | 144.7 | 0.135 | -0.78 |
| LipPEG | 146.6 | 0.185 | -3.09 | 116.9 | 0.135 | -9.87 |
| RA-Lip | 145.6 | 0.105 | -6.50 | 133.0 | 0.160 | -4.50 |
| RA-LipPEG | 136.1 | 0.092 | -10.6 | 119.8 | 0.110 | -11.60 |

Comparing the diameter of liposomes containing retinoic acid or blanks revealed that RA encapsulation does not affect their average diameter, which undergoes a slight reduction in all formulations after the lyophilization process, as shown in Table 2. The polydispersity indices obtained are also within the acceptable range for liposomes (<0.3), indicating monodisperse samples, with the presence of vesicles in a single average size range.

The addition of PEG polymer to the liposome composition leads to a reduction in zeta potential, that is, an increase in negative charge, as shown in Table 2, which is an advantage. In terms of modulus, high zeta potential values are important for good vesicle stability, and in this case, this occurs because PEG radicals, hydrophilic polymers, reduce the hydrophobicity of the particle surface.

Example 3 - Assessment of Liposome Stability Prior to Vaccination

To verify sample stability, liposome sizes were analyzed by DLS in two different experiments immediately after reconstitution in PBS (Figure 1) and again 7 days later (Figure 2). It was observed that the liposomes remained the same size even after 7 days of incubation at 4°C. Corroborating this, it was not observed any yellow precipitate related to the RA released from the samples (Table 3). These results indicated good stability of the liposomes in suspension for at least 7 days at 4°C. In any case, the liposomes were always reconstituted immediately before each vaccine dose.

Table 3: Liposome stability after reconstitution: Mean diameter and PDI.

| | | Day 0 | | Day 7 | |
|---|---|---|---|---|---|
| | | DM (nm) | PDI | DM (nm) | PDI |
| **Lip** | Exp 1 | 167.0 | 0.165 | 172.8 | 0.193 |
| | Exp 2 | 170.9 | 0.195 | 170.7 | 0.194 |
| **Lip-PEG** | Exp 1 | 160.5 | 0.160 | 165.2 | 0.175 |
| | Exp 2 | 141.0 | 0.291 | 147.0 | 0.143 |

Example 4 - RA dosing in Nanoformulations

After disintegration of the liposome lyophilized material, the RA was measured by HPLC. The maximum RA concentration measured was 0.8% (Table 4). This concentration is 20 times higher than the 0.04% quantified in the NLS of the prior art (Bezerra, 2019).

Table 4: RA content in Liposomes and Solid Lipid Nanoparticles (SNLs)

| | RA (% w/w) | SD |
|---|---|---|
| RA-Lip | 0.83 | 0.01 |
| RA-Lip PEG | 0.65 | 0.12 |
| RA-NLS | 0.04 | 0.01 |

Example 5 - Preparation of LaAg Vaccine

*L. amazonensis* promastigotes were washed three times by centrifugation (2500 rpm for 15 min) in PBS to remove proteins from the culture medium. The washed cells were lysed by the addition of sterile double-distilled water and three rapid cycles of freezing in liquid nitrogen and thawing at 30°C. The cell lysate was lyophilized overnight, and the resulting powder was designated LaAg. LaAg was sampled into 25 mg samples and stored at -20°C for up to 36 months without loss of biological activity. For use, 25 mg of LaAg was reconstituted in 1 mL of sterile PBS, quickly vortexed, and used immediately to prepare the vaccination composition.

Example 6 - LaAg Vaccine Protein Measurement

LaAg protein measurement was performed using a commercial BCA kit (Sigma-Aldrich) to determine the protein concentration of the powder. For the analysis, 10 mg of LaAg lyophilized powder was dispersed in 2 mL of PBS, reaching a final concentration of 5 mg/mL. Figure 3 shows the protein concentration of the sample analyzed 12 times.

From this result, it can be concluded that 1978 μg/mL corresponds to the protein in 5000 μg/mL of LaAg reconstituted in PBS, i.e., 40% of the LaAg content is protein. The same result was found using the classic Lowry protein measurement method.

Example 7 - Evaluation of vaccine efficacy comparing the RA-LipPEG adjuvant with other RA adjuvants

The experiment was conducted on female BALB/c mice (n=30), 6-8 weeks old, originated and maintained in the vivarium of the Immunopharmacology Laboratory of the Federal University of Rio de Janeiro in mini-insulators with filtered air, sterilized wood shavings, lighting control to favor the circadian rhythm, and free demand for water and food.

The mice were divided into 6 groups (n=5) and were immunized in an upright position with two doses 7 days apart (Figure 4). Each dose of the vaccine formulations was administered in a final volume of 20 μL (10 μL in each nostril) using a 50 μL single-channel micropipette. The formulations were composed of 10 μg of LaAg protein (LaAg) and 15 μg of RA, either in free form (LaAg+RA) or encapsulated in a conventional liposome (LaAg+RALip) or pegylated (LaAg+RALip-PEG) or encapsulated in solid lipid nanoparticles (SLN). The controls (Placebo) received 20 μL of PBS (10 μL in each nostril). Prior to administration, the size distribution of the liposomes was confirmed by DLS to confirm the stability of the nanosystems.

Seven days after the second dose of the vaccine, the mice were challenged/infected intraocularly with $10^7$ *L. infantum* promastigotes in 100 μL of PBS. For this, *L. infantum* promastigotes (NCL strain) maintained at 26°C in Minimum Essential Medium 199 (MEM 199, Cultilab, Brazil) supplemented with 20% Fetal Bovine Serum (Cultilab, Brazil), antibiotics (100 U/mL penicillin and 100 μg/mL streptomycin, Stem Cell Technologies, USA) and hemin (5 μg/mL, Sigma-Aldrich, USA) were used in the stationary growth phase. Before infection, the parasites were washed 3 times with PBS at 2500 rpm for 15 min to remove serum residues.

Throughout the experiment, the animals' clinical signs were monitored to ensure the absence of adverse reactions resulting from vaccination.

Vaccine efficacy was assessed by determining the parasite load using the Limiting Dilution Assay (LDA) (Figure 5). Thirty days after infection, the mice were euthanized, and their spleens and livers were individually removed, weighed, and macerated in 1 mL and 2 mL of MEM199 medium, respectively. The supernatants from the homogenates (50 μL) were diluted in series (1:3) and in triplicate in 96-well plates with 100 μL of MEM199 + 10% Fetal Bovine Serum.

After 15 days at 26°C, the plates were observed under a light microscope, and the parasite load was calculated according to the equation:

$$\frac{parasites}{organ} = \frac{3^{y-1} \; X \; macerated \; vol.\,(\mu L)}{vol.\,of\; leish.\,added\; in\; 1st\; well\; (= 50\; \mu L)}$$

wherein: y = number of the well where leishmanias were found, assuming that the last dilution containing promastigotes originated from a single amastigote/tissue.

Figure 6 shows the weight of the animals and the spleen and liver after different vaccination protocols followed by infection with *L. infantum,* while Figure 7 shows the parasite load in the spleen and liver of BALB/c mice infected with *L. infantum* after vaccination.

A reduction in the parasite load in the spleen was observed from 7 x $10^7$ (Placebo) to 5 x $10^1$ (LaAg). In the liver, the parasite load decreased from 1.5 x $10^6$ (Placebo) to 7 x $10^1$ (LaAg), an average reduction of 99%.

Regarding the use of the RA adjuvant, the addition of the RA-Lip liposome to the vaccine increased its average efficacy by 76% (spleen and liver). The addition of RA-LipPEG was even more effective, increasing the average efficacy by 97% (spleen and liver), as shown in Figure 7. This result demonstrates that the inclusion of pegylated lipid in the liposome was an advantage in improving RA adjuvanticity. The efficacy of the RA-LipPEG adjuvant of this invention was also compared with the RA-NLS adjuvant from a previous study (BEZERRA et al. 2019) in reducing parasite load. Figure 8 shows that while RA-NLS increased the efficacy of the LaAg vaccine by 65%, RA-LipPEG increased its efficacy by 85%.

The advantage of the complete vaccine (LaAg + RA-LipPEG) was also compared to the commercial LeishTec® vaccine. While the LeishTec® vaccine was 80% effective in reducing bone marrow parasite load compared to placebo, the LaAg + RA-LipPEG vaccine was 97%.

In addition to its greater efficacy, it is important to highlight the greater acceptance of the LaAg + RA-LipPEG vaccine, administered in only two nasal doses, while LeishTec® is administered in three injectable doses.

In addition to the doses, the use of a non-irritating adjuvant stands out in favor of LaAg + RA-LipPEG, while LeishTec® includes saponin as adjuvant, which can induce local and systemic adverse effects (MATIAS et al. 2020).

**References:**

BEZERRA, I. P. S. Efeito adjuvante do ácido retinóico na vacina intranasal LaAg ativa contra a leishmaniose cutânea. Dissertac¸ão de Mestrado. Instituto de Biofísica Carlos Chagas Filho, Universidade Federal do Rio de Janeiro, Rio de Janeiro, Brasil, Agosto de 2012.

BEZERRA, I. P. S. Papel do retinol e do ácido retinóico na leishmaniose cutânea: Efeito na proteção da vacina intranasal LaAg. Tese de Doutorado. Instituto de Biofísica Carlos Chagas Filho, Universidade Federal do Rio de Janeiro, Rio de Janeiro, Brasil, Agosto de 2016.

BEZERRA et al., 2018; BEZERRA I.P.S.; ABIB M.A. AND ROSSI-BERGMANN, B. (2018). Intranasal but not subcutaneous vaccination with Leishmania amazonensis antigens allows early expansion of protective immunity against cutaneous leishmaniasis. Vaccine 36(18): 2480-2486 DOI:10.1016/j.vaccine.2018.03.020.

BEZERRA I.P.S., COSTA-SOUZA B;L.S., CARNEIRO G., FERREIRA L.A.M., GUEDES H.L.M. AND ROSSI-BERGMANN B. Nanoencapsuled retinoic acid as a safe tolerogenic adjuvant for intranasal vaccination against cutaneous leishmaniasis. 2019. Vaccine 36:3660-3667. https://doi.org/10.1016/j.vaccine.2019.05.043

Castro, G. A.; COELHO, A. L.; OLIVEIRA, C. A.; MAHECHA, G. A.; OREFICE, R. L.; FERREIRA, L. A. Formation of ion pairing as an alternative to improve encapsulation and stability and to reduce skin irritation of retinoic acid loaded in solid lipid nanoparticles. International Journal of Pharmaceutics, 381:77-83, 2009.

DA SILVA-COUTO, L.; RIBEIRO-ROMÃO, R. P.; SAAVEDRA, A. F.; DA SILVA COSTA SOUZA, B. L.; MOREIRA, O. C.; GOMES-SILVA, A.; ROSSI-BERGMANN, B.; DA-CRUZ, A. M.; PINTO, E. F. Intranasal vaccination with leishmanial antigens protects golden hamsters (Mesocricetus auratus) against Leishmania (Viannia) braziliensis infection. PLoS Neglected Tropical Diseases, 9:e3439, 2015.;

ALVES, C. P.I. ESTUDO DA ENCAPSULAÇÃO DA ISOTRETINOíNA EM LIPOSSOMAS. Revista Eletrônica de Farmácia, [S. I.], v. 4, n. 1, 2007.

ERKELENS, M. N., & MEBIUS, R. E. (2017). Retinoic Acid and Immune Homeostasis: A Balancing Act. Trends in Immunology, 38(3), 168-180.

LEAL, J. M., MOSQUINI M., COVRE L.P., STAGMILLER N.P., RODRIGUES R.R., CHRISTENSEN D., GUEDES H.L.M., ROSSI-BERGMANN B. and GOMES, D.C.O. (2016) Intranasal vaccination with killed Leishmania amazonensis promastigotes antigen (LaAg) associated with CAF01 adjuvant induces partial protection in BALB/c mice challenged with Leishmania (infantum) chagasi. Parasitology 143(1):1-7, doi:10.1017/S0031182015001250.

LAI SK, WANG YY, HANES J. Mucus-penetrating nanoparticles for drug and gene delivery to mucosal tissues. Adv Drug Deliv Rev. 2009 Feb 27;61(2):158-71. doi: 10.1016/j.addr.2008.11.002.

PINTO, E. F.; CORTEZIA, M. M.; ROSSI-BERGMANN, B. Interferon gamma-inducing oral vaccination with Leishmania amazonensis antigens protects BALB/c and C57BL/6 mice against cutaneous leishmaniasis. Vaccine, 21:3534-3541, 2003.

PINTO, E. F.; PINHEIRO O. R.; RAYOL, A.; LARRAGA, V.; ROSSI-BERGMANN, B. (2004). Intranasal vaccination against cutaneous leishmaniasis using a particulated leishmanial antigen or LACK-DNA. Infection and Immunity, 72:4521-4527, 2004.

PRATTI, J. E. S.; RAMOS, T. D.; PEREIRA, J. C.; FONSECA-MARTINS, A. M.; MACIEL-OLIVEIRA, D.; OLIVEIRA-SILVA, G.; MELO, M. F.; CHAVES, S. P.; GOMES, D. C. O.; DIAZ, B. L.; ROSSI-BERGMANN, B.; GUEDES, H. L. M. Efficacy of intranasal LaAg vaccine against Leishmania amazonensis infection of partially resistant C57BL/6 mice. Parasites and Vectors, 2016.

DINIZ, Danielle Guimarães Almeida; LIMA, Eliana Martins and ANTONIOSI FILHO, Nelson Roberto. Isotretinoína: perfis farmacológico, farmacocinético e analitico. Rev. Bras. Cienc. Farm. [online]., vol.38, n.4 (2002)

MATIAS E.P.; SANTOS F.F.; GUIMARÃES J.P.; CHUCRI T.M. Efeitos adversos da vacina LEISH-TEC® em cães soronegativos para Leishmaniose Visceral / Adverse effects of LEISH-TEC® vaccine on seronegative dogs for Visceral Leishmaniasis. Braz. J. of Develop., 6 (7):53019-53028 (2020)

**Claims**

1. Liposome **characterized by** comprising:

    a) at least one bilayer-forming lipid;
    b) optionally at least one PEGylated lipid; and
    c) at least one retinoid,

wherein the retinoid is incorporated in an amount of 0.1% to 20% w/w of the total liposome.

2. . Liposome, according to claim 1, **characterized in that** the bilayer-forming lipid is chosen from lipids or phospholipids selected from the group of sterols, sphingolipids and/or glycolipids.

3. . Liposome, according to claim 2, **characterized in that** the bilayer-forming lipid is selected from the group comprising cholesterol, sphingomyelins, ceramides, phosphatidylcholines, phosphatidylethanolamine, phosphatidylserines, diacylglycerols, and phosphatidic acids containing one (lyso-) or two (diacyl -) saturated or unsaturated fatty acids with more than 4 carbon atoms and up to 28 carbon atoms.

4. . Liposome, according to claim 3, **characterized in that** the lipid is selected from the group comprising phosphatidic acid (PA), phosphatidylglycerol (PG), phosphatidylethanolamine (PE) and phosphatidylserine (PS), phosphatidylcholine (PC), dipalmitoylphosphatidylcholine (DPPC), distearoylphosphatidylcholine (DSPC), dimiristoylphosphatidylcholine (DMPC), dipalmitoylphosphatidylglycerol (DPPG), distearoylphosphatidylglycerol (DSPG), dimiristoylphosphatidylglycerol (DMPG), dipalmitoylphosphatidic acid (DPP A); distearoylphosphatidic acid (DSP A), dipalmitoylphosphatidylserine (DPPS), dimiristoylphosphatidylserine (DMPS), distearoylphosphatidylserine (DSPS), dipal-mitoylphosphatidylethanolamine (DPPE), dimyristoylphosphatidylethanolamine (DMPE), distearoylphosphatidy-lethanolamine (DSPE), dioleylphosphatidylethanolamine (DOPE), and combinations thereof.

5. . Liposome, according to claim 1, **characterized in that** the optional at least one PEGylated lipid is selected from the group comprising PEG-distearoylphosphatidylethanolamine (DSPE-PEG), PEG-dioleylphosphatidylethanolamine (DOPE-PEG), PEG-ceramides (CER-PEG).

6. . Liposome, according to claim 5, **characterized in that** the optional at least one PEGylated lipid is DSPE-PEG.

7. . Liposome, according to claim 1, **characterized in that** the retinoid is selected from the group comprising retinoic acid (especially all trans-tretinoin retinoic acid), retinyl palmitate, retinol ester, retinol, retinal, isotretinoin, alitretinoin, etretinate, acitretin, tazarotene, bexarotene, adapalene and combinations thereof.

8. . Liposome, according to claim 7, **characterized in that** the retinoid is all trans-tretinoin retinoic acid.

9. . Method of preparing a liposome as defined in any one of claims 1 to 8, **characterized in that** it comprises the steps of:

   a) dissolving the bilayer-forming lipid, the optional PEGylated lipid and the retinoid in chloroform;
   b) forming lipid film by the elimination of solvent;
   c) hydrating the lipid film with NaCl solution containing sucrose; and
   d) extruding through polycarbonate membranes.

10. . Method, according to claim 9, **characterized in that** all of the steps occur protected from light.

11. Method, according to claim 9, **characterized in that** the extrusion of step d) occurs under nitrogen pressure.

12. . Method, according to claim 9, **characterized in that** the dissolution step comprises from 85% w/w to 99.5% w/w of the at least one bilayer forming lipid, from 7% w/w to 12% w/w of the at least one PEGylated lipid and from 0.5% w/w to 6% w/w of the at least one retinoid.

13. . Method, according to claim 9, **characterized by** further comprising an additional step of lyophilizing the liposomes.

14. . Intranasal composition **characterized by** comprising:

   a) from 0.2 μg/μL to 2.0 μg/μL of a liposome comprising a retinoid, wherein the retinoid is incorporated in an amount of 0.1% to 20% w/w of the total liposome;
   b) from 0.2 μg/μL to 3.0 μg/μL of at least one active ingredient; and
   c) a carrier for intranasal administration.

15. . Intranasal composition, according to claim 14, **characterized in that** the liposome comprises a retinoid as defined in any one of claims 1 to 8.

16. . Intranasal composition, according to claim 14, **characterized in that** the active ingredient is an antigen.

17. . Intranasal composition, according to claim 16, **characterized in that** the antigen is a protein, a recombinant protein, DNA, RNA or combinations thereof, from viruses, bacteria, parasite, and/or animal cell including human for which immunity is desired.

18. . Intranasal composition, according to claim 14, **characterized in that** it is administered to an animal.

19. . Intranasal composition, according to claim 18, **characterized in that** the animal is man or dog.

20. . Intranasal composition, according to claim 16, **characterized in that** the antigen is derived from an organism selected from the group comprising *Leishmania donovani, Leishmania pifanoi, Leishmania amazonensis, Leishmania mexican, Leishmania tropica, Leishmania brayanensis, Leishmania guyanensis* and *Leishmania infantum.*

21. . Intranasal composition, according to claim 20, **characterized in that** the antigen is a result of lysis of *L. amazonensis* promastigotes.

22. . Intranasal composition, according to claim 14, **characterized in that** it is for the prevention of leishmaniasis.

23. . Intranasal composition, according to claim 14, **characterized in that** it is for preventing allergies, autoimmune diseases, inflammatory diseases, and/or transplant rejection.

24. . Intranasal composition, according to claim 14, **characterized in that** it is in the form of a lyophilized powder to be reconstituted prior to use.

25. . Intranasal composition, according to claim 24, wherein the reconstitution is immediately prior to use.

26. . Method of preparing intranasal composition as defined in any one of claims 14 to 25, **characterized in that** it comprises the steps of:

    a) obtaining the at least one active ingredient; and
    b) mixing the at least one active ingredient to the liposome in a carrier suitable for intranasal application.

27. . Kit **characterized in that** it comprises:

    a) a lyophile powder for reconstitution; and
    b) a solvent;

wherein the hydrophilic powder comprises:

    1) liposome incorporated retinoid as defined in any one of claims 1 to 8, wherein the retinoid is incorporated in an amount of 0.1% to 20% w/w of the total liposome; and
    2) at least one active ingredient.

28. . Kit, according to claim 27, **characterized by** further comprising instructions for preparing and administering the composition.

29. . Kit, according to claim 27, **characterized by** producing an intranasal composition as defined in any one of claims 14 to 25 when reconstituted.

30. . Use of intranasal composition defined in any one of claims 14 to 25, **characterized in that** it is in the prevention of leishmaniasis.

FIGURE 1

**A) Exp 1 (t = 0):**

Size Distribution RA-Lip

Size Distribution RA-Lip-PEG

**B) Exp 2 (t = 0) :**

Size Distribution RA-Lip

Size Distribution RA-Lip-PEG

## FIGURE 2

### A) Exp 1, t = 7 days.:

Size Distribution RA-Lip

Size Distribution RA-Lip-PEG

1.

2.

### B) Exp 2, t = 7 days.:

Size Distribution RA-Lip

Size Distribution RA-Lip-PEG

3.

4.

# FIGURE 3

# FIGURE 4

FIGURE 5

FIGURE 6

FIGURE 7

**Parasite Load**

## INTERNATIONAL SEARCH REPORT

| | International application No. |
|---|---|
| | **PCT/BR2023/050062** |

**A. CLASSIFICATION OF SUBJECT MATTER**

A61K 9/127 (2006.01)i; A61K 47/60 (2017.01)i; A61K 31/203 (2006.01)i; A61K 39/008 (2006.01)i; A61P 33/02 (2006.01)i
CPC: A61K9/127; A61K47/60; A61K31/203; A61K39/008; A61P33/02

According to International Patent Classification (IPC) or to both national classification and IPC

**B. FIELDS SEARCHED**

Minimum documentation searched (classification system followed by classification symbols)

A61K 9/127; A61K 47/60; A61K 31/203; A61K 39/008; A61P 33/02
CPC: A61K9/127; A61K47/60; A61K31/203; A61K39/008; A61P33/02

Documentation searched other than minimum documentation to the extent that such documents are included in the fields searched

Banco de Patentes Brasileiro, Science Direct, Periódicos Capes

Electronic data base consulted during the international search (name of data base and, where practicable, search terms used)

Espacenet, Patentscope, Derwent, Caplus

**C. DOCUMENTS CONSIDERED TO BE RELEVANT**

| Category* | Citation of document, with indication, where appropriate, of the relevant passages | Relevant to claim No. |
|---|---|---|
| X | WO 8906977 A1 (UNIV TEXAS [US]) 10 August 1989 (1989-08-10)<br>Whole document | 1-13 |
| X | WO 9313751 A1 (UNIV TEXAS [US]) 22 July 1993 (1993-07-22)<br>Claims 7, 9 and 12 | 1-13 |
| X | US 2011200665 A1 (MEI XINGGUO,) 18 August 2011 (2011-08-18)<br>Claims 1, 6 and 9 | 1-13 |
| X | EP 3501500 A1 (UNIV SHANGHAI JIAOTONG [CN]) 26 June 2019 (2019-06-26)<br>Claims 1, 6 and 8 | 1-13 |
| X | WO 2008116135 A2 (PATHFINDER MAN INC [US]) 25 September 2008 (2008-09-25)<br>Claims 1 and 5 | 1-13 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **04 April 2023** | **12 April 2023** |

| Name and mailing address of the ISA/BR | Authorized officer |
|---|---|
| **Instituto Nacional da Propriedade Industrial (Brasil)**<br>**Rua Mayrink Veiga, 9, 6° andar, CEP 20.090-910 Rio de Janeiro – RJ**<br>**Brazil** | **Luiz Eduardo Kaercher** |
| Telephone No. **(55 21) 3037-3742, 3037-3984** | Telephone No. **552130374528** |

Form PCT/ISA/210 (second sheet) (July 2022)

**EP 4 670 711 A1**

## INTERNATIONAL SEARCH REPORT

| | International application No. |
| --- | --- |
| | **PCT/BR2023/050062** |

**C.    DOCUMENTS CONSIDERED TO BE RELEVANT**

| Category* | Citation of document, with indication, where appropriate, of the relevant passages | Relevant to claim No. |
| --- | --- | --- |
| A | Izabella Pereira Silva Bezerra, Marina Amaral Abib, Bartira Rossi-Bergmann, Intranasal but not subcutaneous vaccination with LaAg allows rapid expansion of protective immunity against cutaneous leishmaniasis, Vaccine, Volume 36, Issue 18, 25 April 2018, Pages 2480-2486. | 14-30 |
| A | Izabella P.S. Bezerra, Beatriz L.S. Costa-Souza, Guilherme Carneiro, Lucas Antonio Miranda Ferreira, Herbert Leonel de Matos Guedes, Bartira Rossi-Bergmann, Nanoencapsulated retinoic acid as a safe tolerogenic adjuvant for intranasal vaccination against cutaneous leishmaniasis, Vaccine, Volume 37, Issue 28, 19 June 2019, Pages 3660-3667. | 14-30 |
| A | H. Naseri, F. Eskandari, M. R. Jaafari, A. Khamesipour, A. Abbasi, A. Badiee, PEGylation of cationic liposomes encapsulating soluble Leishmania antigens reduces the adjuvant efficacy of liposomes in murine model, Parasite Immunology. 2017;39:e12492. | 14-30 |

Form PCT/ISA/210 (second sheet) (July 2022)

19

**INTERNATIONAL SEARCH REPORT**
Information on patent family members

International application No.

**PCT/BR2023/050062**

| Patent document cited in search report | | | Publication date (day/month/year) | Patent family member(s) | | | Publication date (day/month/year) |
|---|---|---|---|---|---|---|---|
| WO | 8906977 | A1 | 10 August 1989 | EP | 0397774 | A1 | 22 November 1990 |
| WO | 9313751 | A1 | 22 July 1993 | AT | 173615 | T | 15 December 1998 |
| | | | | AU | 3442093 | A | 03 August 1993 |
| | | | | CA | 2128103 | A1 | 22 July 1993 |
| | | | | CA | 2128103 | C | 28 December 2004 |
| | | | | DE | 69322252 | D1 | 07 January 1999 |
| | | | | DE | 69322252 | T2 | 12 August 1999 |
| | | | | EP | 0621773 | A1 | 02 November 1994 |
| | | | | EP | 0621773 | B1 | 25 November 1998 |
| | | | | ES | 2128417 | T3 | 16 May 1999 |
| | | | | JP | H07503014 | A | 30 March 1995 |
| | | | | JP | 3691054 | B2 | 31 August 2005 |
| | | | | JP | 2005232182 | A | 02 September 2005 |
| US | 2011200665 | A1 | 18 August 2011 | US | 8642074 | B2 | 04 February 2014 |
| | | | | AU | 2008323514 | A1 | 22 May 2009 |
| | | | | AU | 2008323514 | A2 | 17 May 2012 |
| | | | | AU | 2008323514 | B2 | 12 June 2014 |
| | | | | BR | PI0820505 | A2 | 16 June 2015 |
| | | | | CA | 2704258 | A1 | 22 May 2009 |
| | | | | CA | 2704258 | C | 22 November 2016 |
| | | | | CN | 101883557 | A | 10 November 2010 |
| | | | | CN | 101883557 | B | 29 May 2013 |
| | | | | EP | 2217209 | A1 | 18 August 2010 |
| | | | | EP | 2217209 | A4 | 25 September 2013 |
| | | | | EP | 2217209 | B1 | 06 June 2018 |
| | | | | ES | 2689259 | T3 | 12 November 2018 |
| | | | | HK | 1146906 | A1 | 22 July 2011 |
| | | | | JP | 2011502134 | A | 20 January 2011 |
| | | | | JP | 5570994 | B2 | 13 August 2014 |
| | | | | KR | 20100087030 | A | 02 August 2010 |
| | | | | KR | 101652126 | B1 | 29 August 2016 |
| | | | | MX | 2010004957 | A | 02 August 2010 |
| | | | | MY | 151268 | A | 30 April 2014 |
| | | | | RU | 2010123009 | A | 20 December 2011 |
| | | | | RU | 2497499 | C2 | 10 November 2013 |
| | | | | TW | 200930380 | A | 16 July 2009 |
| | | | | TW | I437995 | B | 21 May 2014 |
| | | | | WO | 2009059449 | A1 | 14 May 2009 |
| | | | | WO | 2009062398 | A1 | 22 May 2009 |
| EP | 3501500 | A1 | 26 June 2019 | EP | 3501500 | A4 | 24 June 2020 |
| | | | | EP | 3501500 | B1 | 11 May 2022 |
| | | | | CN | 107753427 | A | 06 March 2018 |
| | | | | CN | 107753427 | B | 14 January 2022 |
| | | | | ES | 2919999 | T3 | 29 July 2022 |
| | | | | US | 2021283086 | A1 | 16 September 2021 |
| | | | | WO | 2018033118 | A1 | 22 February 2018 |
| WO | 2008116135 | A2 | 25 September 2008 | WO | 2008116135 | A3 | 24 December 2008 |
| | | | | AU | 2008228764 | A1 | 25 September 2008 |
| | | | | AU | 2008228764 | B2 | 08 May 2014 |
| | | | | AU | 2014202278 | A1 | 22 May 2014 |

Form PCT/ISA/210 (patent family annex) (July 2022)

## INTERNATIONAL SEARCH REPORT
### Information on patent family members

| | International application No. |
|---|---|
| | **PCT/BR2023/050062** |

| Patent document cited in search report | Publication date (day/month/year) | Patent family member(s) | | | Publication date (day/month/year) |
|---|---|---|---|---|---|
| | | AU | 2014202278 | B2 | 02 June 2016 |
| | | AU | 2014202278 | C1 | 13 April 2017 |
| | | BR | PI0809164 | A2 | 16 September 2014 |
| | | BR | PI0809164 | B1 | 04 August 2020 |
| | | BR | PI0809164 | B8 | 28 February 2023 |
| | | CA | 2680825 | A1 | 25 September 2008 |
| | | CA | 2680825 | C | 29 October 2013 |
| | | CA | 2823407 | A1 | 25 September 2008 |
| | | CA | 2823407 | C | 18 October 2016 |
| | | EP | 2136787 | A2 | 30 December 2009 |
| | | EP | 2136787 | A4 | 31 July 2013 |
| | | EP | 2136787 | B1 | 21 August 2019 |
| | | EP | 3607937 | A1 | 12 February 2020 |
| | | ES | 2755840 | T3 | 23 April 2020 |
| | | MX | 2009010170 | A | 26 November 2009 |
| | | MX | 337408 | B | 03 March 2016 |
| | | MX | 352434 | B | 24 November 2017 |
| | | MX | 363435 | B | 22 March 2019 |
| | | NO | 20093032 | L | 22 October 2009 |
| | | US | 2008233183 | A1 | 25 September 2008 |
| | | US | 8454945 | B2 | 04 June 2013 |
| | | US | 2013202683 | A1 | 08 August 2013 |
| | | US | 10588859 | B2 | 17 March 2020 |
| | | US | 2021015748 | A1 | 21 January 2021 |
| | | ZA | 201400071 | B | 23 December 2014 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018078053 A
- WO 2006110915 A

- EP 2068918 A

**Non-patent literature cited in the description**

- *Vaccine*, vol. 36 (18), 2480-2486
- **BEZERRA I.P.S.** ; **COSTA-SOUZA B** ; **L.S., CARNEIRO G.** ; **FERREIRA L.A.M.** ; **GUEDES H.L.M.** ; **ROSSI-BERGMANN B.** *Vaccine*, 2019, vol. 36, 3660-3667
- **CASTRO, G. A.** ; **COELHO, A. L.** ; **OLIVEIRA, C. A.** ; **MAHECHA, G. A.** ; **OREFICE, R. L.** ; **FERREIRA, L. A.** *International Journal of Pharmaceutics*, 2009, vol. 381, 77-83
- **DA SILVA-COUTO, L.** ; **RIBEIRO-ROMÃO, R. P.** ; **SAAVEDRA, A. F.** ; **DA SILVA COSTA SOUZA, B. L.** ; **MOREIRA, O. C.** ; **GOMES-SILVA, A.** ; **ROSSI-BERGMANN, B.** ; **DA-CRUZ, A. M.** ; **PINTO, E. F.** Leishmania (Viannia) braziliensis infection.. *PLoS Neglected Tropical Diseases*, 2015, vol. 9, e3439
- **ALVES, C. P.I.** ESTUDO DA ENCAPSULAÇÃO DA ISOTRETINOíNA EM LIPOSSOMAS.. *Revista Eletrônica de Farmácia, [S. I.*, 2007, vol. 4 (1)
- **ERKELENS, M. N.** ; **MEBIUS, R. E.** Retinoic Acid and Immune Homeostasis: A Balancing Act.. *Trends in Immunology*, 2017, vol. 38 (3), 168-180
- **LEAL, J. M.** ; **MOSQUINI M.** ; **COVRE L.P.** ; **STAGMILLER N.P.** ; **RODRIGUES R.R.** ; **CHRISTENSEN D.** ; **GUEDES H.L.M.** ; **ROSSI-BERGMANN B.** ; **GOMES, D.C.O.** *Parasitology*, 2016, vol. 143 (1), 1-7

- **LAI SK** ; **WANG YY** ; **HANES J.** Mucus-penetrating nanoparticles for drug and gene delivery to mucosal tissues.. *Adv Drug Deliv Rev.*, 27 February 2009, vol. 61 (2), 158-71
- **PINTO, E. F.** ; **CORTEZIA, M. M.** ; **ROSSI-BERGMANN, B.** Leishmania amazonensis antigens protects BALB/c and C57BL/6 mice against cutaneous leishmaniasis.. *Vaccine*, 2003, vol. 21, 3534-3541
- **PINTO, E. F.** ; **PINHEIRO O. R.** ; **RAYOL, A.** ; **LARRAGA, V.** ; **ROSSI-BERGMANN, B.** *Infection and Immunity*, 2004, vol. 72, 4521-4527
- **PRATTI, J. E. S.** ; **RAMOS, T. D.** ; **PEREIRA, J. C.** ; **FONSECA-MARTINS, A. M.** ; **MACIEL-OLIVEIRA, D.** ; **OLIVEIRA-SILVA, G.** ; **MELO, M. F.** ; **CHAVES, S. P.** ; **GOMES, D. C. O.** ; **DIAZ, B. L.** Leishmania amazonensis infection of partially resistant C57BL/6 mice.. *Parasites and Vectors*, 2016
- **DINIZ, DANIELLE GUIMARÃES ALMEIDA** ; **LIMA, ELIANA MARTINS** ; **ANTONIOSI FILHO** ; **NELSON ROBERTO**. Isotretinoína: perfis farmacológico, farmacocinético e analitico.. *Rev. Bras. Cienc. Farm. [online].*, 2002, vol. 38 (4)
- **MATIAS E.P.** ; **SANTOS F.F.** ; **GULMARÃES J.P.** ; **CHUCRI T.M.** Leishmaniasis.. *Braz. J. of Develop.*, 2020, vol. 6 (7), 53019-53028